# EUROPEAN PATENT SPECIFICATION

(11) **EP 4 280 973 B1**
(45) Date of publication and mention of the grant of the patent: **20.05.2026**
(21) Application number: 22743243.2
(22) Date of filing: 21.01.2022
(51) Int. Cl.: A61B 17/04, A61F 2/08, A61B 17/00

(54) **SOFT TISSUE IMPLANTS AND INSTRUMENTATION**
WEICHGEWEBEIMPLANTATE UND INSTRUMENTE
IMPLANTS DE TISSU MOU ET INSTRUMENTATION

(30) Priority: 22.01.2021 US 202163140596 P
(43) Date of publication of application: 29.11.2023
(73) Proprietor: Paragon 28, Inc., Englewood, CO 80112 (US)
(72) Inventor: DACOSTA, Albert, Englewood, Colorado 80112 (US); MAJORS, Benjamin, Englewood, Colorado 80112 (US); DEVASCONCELLOS, Paul, Englewood, Colorado 80112 (US); HARTSON, Kyle, James, Englewood, Colorado 80112 (US); ALLARD, Randy, Golden, Colorado 80401 (US)
(74) Representative: Murgitroyd & Company
(86) International application number: PCT/US2022/013302
(87) International publication number: WO 2022/159695

(56) References cited:
- US-A1- 2008 033 460
- US-A1- 2015 142 052
- US-A1- 2017 135 688
- US-A1- 2019 167 252
- US-A1- 2019 336 190
- US-A1- 2019 336 270
- US-A1- 2020 022 693
- US-A1- 2020 022 693
- US-A1- 2020 155 140
- US-B2- 10 441 450

## Description

### FIELD

The present disclosure relates to soft tissue implant systems, instruments, and related methods. The present disclosure relates to podiatric and orthopedic implants and surgery related to repairs of soft tissue and/or bone. More specifically, but not exclusively, the present disclosure relates to instruments, implants, devices, systems, assemblies, and methods for joining soft tissue to soft tissue, soft tissue to bone, and bone to bone.

### BACKGROUND OF THE INVENTION

Many currently available implants, instrumentation, systems, and methods for addressing soft tissue trauma (acute and chronic, e.g., defect, gradual deterioration, etc.) do not completely address the needs of patients. Additionally, many currently available implants, instrumentation, systems, and methods for addressing soft tissue trauma fail to account for properties of soft tissue anatomy and further require healthcare providers to perform steps that can reduce the efficiency of the procedure as well as the favorability of the outcome for the patient. US2017/135688A1, US2020/022693A1, US2008/033460A1 and US2020/155140A1 disclose related art.

US2020/155140A1 discloses an anchoring system including a suture anchor and an implant delivery device. The suture anchor includes an anchor body and a distal member that is rotatable relative to the anchor body about a longitudinal suture anchor axis. The distal member has an aperture that traverses the distal member and includes a slot and an eyelet, the eyelet being sized to permit a suture to traverse the distal member through the eyelet. The implant delivery device includes a handle portion, a drive shaft portion including a cannulated outer shaft, and a suture pulley shaft including an inner shaft. The cannulated outer shaft is configured to engage a proximal end of the anchor body for driving the suture anchor into a bore. The inner shaft is slidably received in the cannulated outer shaft to maintain the position and tension of the sutures during insertion.

### SUMMARY

The invention is defined by appended claim 1.
The present disclosure is directed toward soft tissue implants, implant systems, instruments, and related methods.

A first aspect of the present disclosure is a system for repairing or reconstructing soft tissue. The system includes includes an instrument having a handle and an extension, the extension opposite the handle, an implant releasably engaged with the instrument, and a suture threaded through at least a portion of the implant, wherein the suture is engaged with a tensioning mechanism of the instrument.

According to the first aspect of the present disclosure, the implant includes a top portion, an bottom portion, and a body portion disposed between the top portion and the bottom portion, wherein the top portion, the bottom portion, and the body portion are arranged about a common longitudinal axis.

According to the first aspect of the present disclosure, the implant is cannulated through the top portion and at least a portion of at least one of the body portion and the bottom portion.

According to the first aspect of the present disclosure, the implant includes least one vent disposed along an outer surface of at least one of the top portion and the body portion, wherein the at least one vent establishes fluid communication between the outer surface and an inner surface the implant.

According to the first aspect of the present disclosure, the implant includes a threading disposed along at least a portion of the outer surface of the top portion and the body portion, wherein the threading includes a plurality of threads equidistantly spaced from one another.

According to the first aspect of the present disclosure, the threading is configured to span at least a portion of the at least one vent.

According to the first aspect of the present disclosure, the implant includes at least one tap arranged on an outer surface of the bottom portion of the implant.

According to the first aspect of the present disclosure, the at least one tap includes a plurality of taps arranged about the outer surface of the bottom portion of the implant, wherein the plurality of taps protrude from the outer surface and are equidistantly spaced from one another.

According to the first aspect of the present disclosure, the implant is releasably couplable with a tip of the extension of the implant.

According to the first aspect of the present disclosure, the instrument comprises a tensioning mechanism disposed within a housing of the instrument.

According to the first aspect of the present disclosure, the tensioning mechanism includes a first arm, a second arm, and a resilient member.

According to the first aspect of the present disclosure, the second arm is pivotably coupled with the first arm.

According to the first aspect of the present disclosure, the instrument includes a track disposed on at least a portion of the outer surface of the handle, wherein the track is configured to receive at least a portion of a suture at least partially therein.

According to the first aspect of the present disclosure, the second arm includes an engagement portion configured to engage with at least a portion of the suture, wherein the engagement portion is arranged adjacent at least a portion of the track.

According to the first aspect of the present disclosure, the resilient member is a spring.

According to the first aspect of the present disclosure, actuation of at least one of the first arm and the second arm compresses the spring.

A second aspect of the present disclosure includes an instrument. The instrument includes a handle having a housing. The housing includes a tensioning mechanism having a first arm, a second arm pivotably coupled with the first arm, and a resilient member. The handle also includes a track disposed on at least a portion of an outer surface of the housing, wherein the track is configured to receive at least a portion of a suture. The instrument also includes an extension configured to releasably couple with an implant.

According to the second aspect of the present disclosure, the second arm includes an engagement portion configured to releasably engage with at least a portion of the suture such that actuation of the second arm engages and disengages the engagement portion with at least a portion of the suture.

A third aspect of the present disclosure includes an implant. The implant includes an upper portion with at least one vent disposed in an outer surface of the upper portion and establishing fluid communication between the outer surface and an inner surface of the upper portion. The implant also includes a threading disposed on the outer surface of the upper portion, wherein the threading is configured to span the at least one vent. The implant also includes an bottom portion, with the upper portion and the bottom portion having a substantially cylindrical geometry and are arranged concentrically about a common longitudinal axis.

According to the third aspect of the present disclosure, at least the upper portion of the implant is cannulated.

### BRIEF DESCRIPTION OF THE DRAWINGS

The accompanying drawings, which are incorporated in and constitute a part of the specification, illustrate embodiments of the disclosure and together with the detailed description herein, serve to explain the principles of the inventions. It is emphasized that, in accordance with the standard practice in the industry, various features may or may not be drawn to scale. In fact, the dimensions of the various features may be arbitrarily increased or reduced for clarity of discussion. The drawings are only for purposes of illustrating embodiments of the disclosure and are not to be construed as limiting the inventions.
**FIG. 1** is a side perspective view of an exemplary implant and instrument for facilitating soft tissue repair and/or reconstruction in conjunction with an implant system, in accordance with the present disclosure;
**FIG. 2** is a side perspective view of an exemplary implant and instrument for facilitating soft tissue repair and/or reconstruction in conjunction with an implant system, in accordance with the invention;
**FIG. 3** is an end perspective view of the implant of FIG. 2 for facilitating soft tissue repair and/or reconstruction in conjunction with an implant system, in accordance with the present disclosure;
**FIG. 4** is bottom perspective view of the exemplary implant of FIG. 2 facilitating soft tissue repair and/or reconstruction in conjunction with an implant system, in accordance with the present disclosure;
**FIG. 5** is a side perspective view of the exemplary implant of FIG. 2 and an additional instrument for facilitating soft tissue repair and/or reconstruction in conjunction with an implant system, in accordance with the present disclosure;
**FIG. 6** is a perspective view of the implants and instruments of FIG. 1 and FIG. 5, in accordance with the present disclosure;
**FIG. 7** is a side perspective view of an exemplary implant for facilitating soft tissue repair and/or reconstruction in conjunction with an implant system, in accordance with the present disclosure;
**FIG. 8** is bottom perspective view of the exemplary implant of FIG. 7 for facilitating soft tissue repair and/or reconstruction in conjunction with an implant system, in accordance with the present disclosure;
**FIG. 9** is a side perspective view of an exemplary implant for facilitating soft tissue repair and/or reconstruction in conjunction with an implant system, in accordance with the present disclosure;
**FIG. 10** is bottom perspective view of the exemplary implant of FIG. 9 and instrument for facilitating soft tissue repair and/or reconstruction in conjunction with an implant system, in accordance with the present disclosure;
**FIG. 11** is a side view of an exemplary instrument for facilitating soft tissue repair and/or reconstruction in conjunction with an implant system, in accordance with the present disclosure;
**FIG. 12** is a side cross-sectional view of the exemplary instrument of FIG. 11 for facilitating soft tissue repair and/or reconstruction in conjunction with an implant system, in accordance with the present disclosure;
**FIG. 13** is a perspective view of a portion of the exemplary instrument of FIGS. 11-12 for facilitating soft tissue repair and/or reconstruction in conjunction with an implant system, in accordance with the present disclosure
**FIG. 14** is a side view of an exemplary instrument for facilitating soft tissue repair and/or reconstruction in conjunction with an implant system, in accordance with the present disclosure;
**FIG. 15** is a side, front, perspective view of the exemplary instrument of FIG. 14 for facilitating soft tissue repair and/or reconstruction in conjunction with an implant system, in accordance with the present disclosure;
**FIG. 16** is a side, cross-sectional view of the exemplary instrument of FIG. 14 for facilitating soft tissue repair and/or reconstruction in conjunction with an implant system, in accordance with the present disclosure;
**FIG. 17** is a cutaway top view of the exemplary instrument of FIG. 14 for facilitating soft tissue repair and/or reconstruction in conjunction with an implant system, in accordance with the present disclosure;
**FIG. 18** is a front perspective view of an exemplary implant for facilitating soft tissue repair and/or reconstruction in conjunction with an implant system, in accordance with the present disclosure; and
**FIG. 19** is a side view of the exemplary implant of FIG. 18 for facilitating soft tissue repair and/or reconstruction in conjunction with an implant system, in accordance with the present disclosure.

### DETAILED DESCRIPTION

In this detailed description and the following claims, the words proximal, distal, anterior or plantar, posterior or dorsal, medial, lateral, superior and inferior are defined by their standard usage for indicating a particular part or portion of a bone or implant according to the relative disposition of the natural bone or directional terms of reference. For example, "proximal" means the portion of a device or implant nearest the torso, while "distal" indicates the portion of the device or implant farthest from the torso. As for directional terms, "anterior" is a direction towards the front side of the body, "posterior" means a direction towards the back side of the body, "medial" means towards the midline of the body, "lateral" is a direction towards the sides or away from the midline of the body, "superior" means a direction above and "inferior" means a direction below another object or structure. Further, specifically in regards to the foot, the term "dorsal" refers to the top of the foot and the term "plantar" refers the bottom of the foot.

Similarly, positions or directions may be used herein with reference to anatomical structures or surfaces. For example, as the current implants, devices, instrumentation, and methods are described herein with reference to use with the bones of the foot, the bones of the foot, ankle and lower leg may be used to describe the surfaces, positions, directions or orientations of the implants, devices, instrumentation and methods. Further, the implants, devices, instrumentation, and methods, and the aspects, components, features and the like thereof, disclosed herein are described with respect to one side of the body for brevity purposes. However, as the human body is relatively symmetrical or mirrored about a line of symmetry (midline), it is hereby expressly contemplated that the implants, devices, instrumentation, and methods, and the aspects, components, features and the like thereof, described and/or illustrated herein may be changed, varied, modified, reconfigured or otherwise altered for use or association with another side of the body for a same or similar purpose without departing from the scope of the invention. For example, the implants, devices, instrumentation, and methods, and the aspects, components, features and the like thereof, described herein with respect to the right foot may be mirrored so that they likewise function with the left foot. Further, the implants, devices, instrumentation, and methods, and the aspects, components, features and the like thereof, disclosed herein are described with respect to the foot for brevity purposes, but it should be understood that the implants, devices, instrumentation, and methods may be used with other bones of the body having similar structures.

The instruments, implants, systems, assemblies, and related methods for repairing, reconstructing, or facilitating the repair/reconstruction of the present disclosure may be similar to, such as include at least one feature or aspect of, the implants, systems, assemblies and related methods disclosed in International PCT Application No. PCT/US2020/070866, filed on December 4, 2020, and entitled Soft Tissue Repair; U.S. Provisional Application No. 62/968,965, filed January 31, 2020, and entitled Knotless Soft Tissue Implant Systems and Related Methods; and/or U.S. Provisional Application No. 63/034,066 filed on June 3, 2020, entitled Soft Tissue Implant Systems, Instruments, and Related Methods. Similarly, the instruments, implants, systems, assemblies, and related methods for maintaining, correcting, and/or resurfacing joint surfaces of the present disclosure may include one or more instrument (e.g., one or more insertion and/or implantation instruments) disclosed in International PCT Application No. PCT/US2020/070866, filed on December 4, 2020, and entitled Soft Tissue Repair; U.S. Provisional Application No. 62/968,965, filed January 31, 2020, and entitled Knotless Soft Tissue Implant Systems and Related Methods; and/or U.S. Provisional Application No. 63/034,066 filed on June 3, 2020, entitled Soft Tissue Implant Systems, Instruments, and Related Methods.

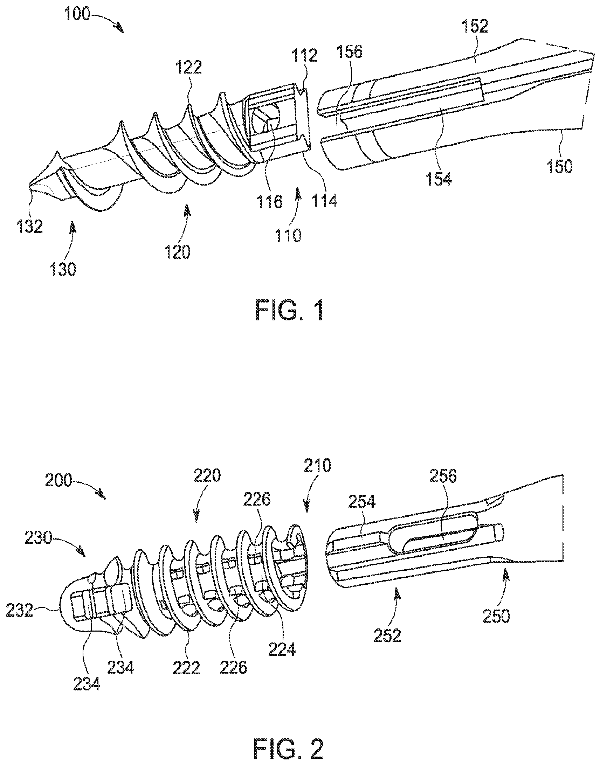

Referring to the drawings, wherein like reference numerals are used to indicate like or analogous components throughout the several views, and with particular reference to FIG. 1, there is illustrated an exemplary embodiment of an implant 100 (e.g., anchor, etc.) for fixation within bone and/or soft tissue so as to facilitate repair and/or reconstruction of soft tissue. Also shown in FIG. 1 is an instrument 150 configured to engage with and/or facilitation the implantation, manipulation, adjustment, and/or removal of the implant 100. The implant 100 is shown to include a top portion 110 and a bottom portion 130, with a body portion 120 disposed between the top portion 110 and the bottom portion 130. The top portion 120 of the implant 100 is shown to include a head 112 arranged at the proximal end of the top portion of the implant 100. As shown in the exemplary embodiment of FIG. 1, the head 112 includes an interface feature 114 positioned at the proximal end of the top portion 110. The interface feature 114 is configured to engage (e.g., interface, releasably couple with, receive a portion of, etc.) with at least a portion of the instrument 150. The instrument 150 is shown to include an interface feature 154 arranged on a distal portion 152 of the instrument 150, with the interface feature 154 having a complimentary geometry to that of the interface feature 114 of the implant 100. Further, the instrument 150 is shown to include a recess 156 arranged on the distal portion 152 of the instrument 150. The recess 156 is shown to include at least a portion of the substantially cylindrical geometry of the distal portion 152 and may be configured to align with one or more portions of the implant 100 to facilitate threading of one or more needles and/or sutures. As shown in the exemplary embodiment, of FIG. 1, the interface feature 114 is shown to have a hexalobe geometry. In alternate embodiments, the interface feature 114 may have alternate geometries, for example a traditional torx geometry, a hexagonal geometry or various geometries with one or more splines arranged variously about the interface feature 114. Accordingly, such alternate geometries of the interface feature 114 may be configured to accommodate a portion of one or more instruments as mentioned previously, for example a driver or other instrument.

The top portion 110 is further shown to include an opening (e.g., an aperture, eyelet, etc.) 116 configured to receive a suture threaded through said opening 116. As shown in the exemplary embodiment of FIG. 1, the opening 116 is defined in lateral directions by at least a portion of the interface feature 114, and in a distal direction by at least a portion of the body portion 120. The opening 116 may be arranged such that when the implant 114 interfaces with an instrument such as those previously mentioned, the opening 116 is aligned with and/or positioned adjacent one or more openings of said instruments (e.g., the recess 156 of the instrument 150). For example, and instrument may include a shaft with diametrically opposed openings with which the opening 116 may align when the implant 100 engages with the instrument via at least the interface feature 114. The alignment of the openings of the instrument with the opening 116 of the implant 100 may be configured to facilitate threading of a suture through one opening of the instrument, through the opening 116 of the implant 100 engaged with the instrument, and subsequently through a second opening of the instrument. Such threading of a suture through the opening 116 engages the suture with the implant 100 prior to implantation and further allows for the implant 100 to be implanted within bone and/or soft tissue without requiring an intermediate step to thread the suture after implantation has begun. Following implantation, the instrument may be disengaged from the interface feature 114 of the implant 100 and the suture, with the suture then free to be secured and/or manipulated as needed (e.g., coupled with one or more other implants, tensioned, etc.).

The body portion 120 of the implant 100 is shown to include a threading 122 arranged along a length of the body portion 120. In some embodiments, the threading 122 may extend proximally along the body portion 120 such that the threading terminates adjacent and/or abuts the interface feature 114 of the top portion 110. Further, the threading 122 may further extend distally such that the threading 122 terminates at a distal portion of the bottom portion 130 of the implant 100. The threading 122 is shown to extending circumferentially around the implant 100 while extending continuously from the bottom portion 130 to the top portion 110. As shown in the exemplary embodiment of FIG. 1, the threading 122 is a dual-lead thread while in alternate embodiments, the threading 122 may be a single-lead threading. In some embodiments, the threading 122 may include alternate configurations and/or lengths. The threading 122 is shown to include substantially equivalent spacing (e.g., distance between the threads in the proximal-distal direction when viewed from a side view such as that shown in FIG. 1) along the body portion 120 of the implant 100, with the threading 122 having a greater spacing on the bottom portion 130 of the implant 100. In some embodiments, the threading 122 may have equivalent spacing along the length of the implant 100, whereas in other embodiments the threading 122 may have one or more different spacing intervals along the length of the implant 100. The threading 122 may terminate in a tip portion 132 of the bottom portion 130 as shown in the exemplary embodiment of FIG. 1. The tip portion 132 may be configured to facilitate implantation of the implant 100 into bone and/or soft tissue such that the tip portion 132 facilitates entry of the implant into said bone and/or soft tissue. Further, as the implant 100 is implanted in bone and/or soft tissue, soft tissue may be pulled in the direction in which the implant 100 is inserted (e.g., into the bone) so as to achieve desired placement of soft tissue from a displaced position. Once the implant 100 is implanted, the implant 100 may be disengaged from any instrument implemented in implantation of the implant 100 and the suture may be tied off and/or coupled with other adjacent implants, anatomy, or sutures.

The implant 100 may have one or more standard lengths, with said length measured from the proximal-most point of the top portion 110 to the distal-most portion of the bottom portion 130. For example, the implant 100 may have a length ranging from 2 mm to 6mm, with various different sized produced and/or provided in a surgical kit in increments of 0.5 mm. Additionally, the embodiment of the implant 100 shown in FIG. 1 may be available in multiple lengths (e.g., as components of a surgical kit or independently) so as to accommodate different applications of implantation into bone and/or soft tissue to facilitate repair and/or reconstruction of said soft tissue. Further, the implant 100 may have a standard diameter and/or may be available in various different diameters so as to accommodate different applications of implantation into bone and/or soft tissue to facilitate repair and/or reconstruction of said soft tissue. Such various diameters may be components of a surgical kit (e.g., a sterile kit including multiple lengths and diameters of the implant 100 as well as other components, for example one or more k-wires, drills, and/or drivers) and/or may be available independently of such a kit. The implant 100 may preferably be titanium, although the implant 100 may also be available in PEEK and/or other polymeric soft anchor configurations. In some aspects, the implant 100 may be produced and/or provided with a suture (e.g., those shown and described in the patent applications referenced previously, or other similar sutures and/or suture tapes) threaded through the opening 116. Further, in some aspects the implant 100 may be produced and/or provided (e.g., in a sterile surgical kit) in a pre-loaded state, where the implant 100 is engaged with an instrument via the interface feature 114 and a suture as described previously threaded through the implant 100 and at least a portion of said instrument.

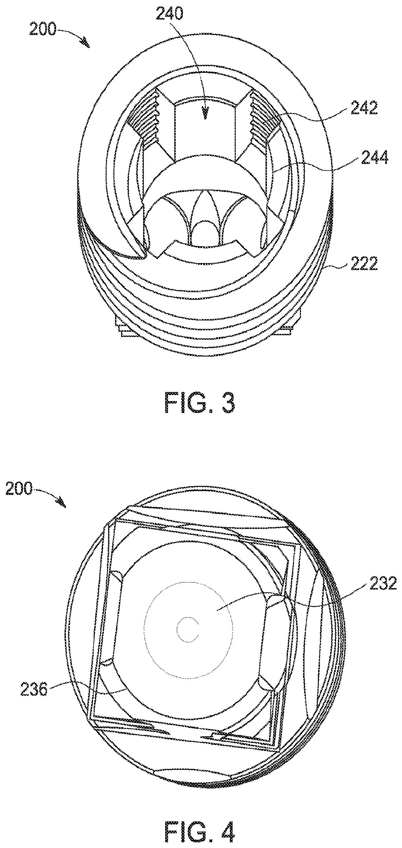

Referring now to FIGS. 2-4, an exemplary embodiment of an implant 200 is shown. The implant 200 is shown to include a top portion 210, a bottom portion 230, and a body portion 220 arranged between the top portion 210 and the bottom portion 230. The implant 200 is further shown to include a channel (e.g., a cannulated opening) 240 extending along a longitudinal axis of the implant 200 from the proximal-most point of the top portion 210 through the body portion 220 and terminating at the distal-most point of the body portion 220. The implant 200 is shown to include an interface feature 242 arranged within the channel 240 and, as shown in the exemplary embodiment of FIGS. 2-4, disposed circumferentially about an inner surface 244 of the implant 200. The interface feature 242 of exemplary embodiment of the implant 200 shown in FIGS. 2-4 includes four splines arranged circumferentially about the inner surface 244 of the implant 200 and extending along the inner surface 244 from the top portion 210 of the implant in a distal direction, with each of said splines positioned in approximately 90-degree increments (e.g., the splines are equally spaced circumferentially about the inner surface 244). In some embodiments, the interface feature 242 may include a greater or lesser number of splines, for example one, two, three, five, or another alternate number of splines. Further, the splines of the interface feature 244 may be arranged variously about the inner surface 244 of the implant 200, for example in some embodiments the splines may not have approximately equal spacing from one another as shown and described in FIG. 3. In some embodiments, the splines may include a geometry other than that shown and described with reference to FIG. 3, for example the splines may include a substantially triangular geometry.

The interface feature 242 is configured to engage (e.g., receive, releasably couple with, etc.) one or more instruments that may be provided in a surgical kit along with the implant 200. As shown in FIG. 2, an instrument (e.g., a driver) 250 is shown to include an interface feature 254 arranged on a distal portion 252 of the instrument 250. The interface feature 254 of the instrument 250 is shown to be complimentary (e.g., geometrically opposite) to that of the interface feature 242. As shown in FIG. 2, the interface feature 254 of the instrument 250 includes four splines complimentary to those of the interface feature 242 of the implant 200. In some embodiments, the complimentary four splines of the interface features 242, 254 may be configured to accommodate greater torque than other similar geometries (e.g., torque from the instrument 250 being applied to the implant 200 so as to implant the implant 200 in bone and/or soft tissue). The distal portion 252 of the implant 250 may be received (e.g., to engage, releasably couple, etc.) within the channel 240 of the implant 200 such that the interface feature 254 engages with the interface feature 242 along the at least a portion of the length of the interior surface 244 of the channel 240. The instrument 250 is further shown to include an opening 256 arranged on the distal portion 252 thereof, with said opening 256 configured such that it may align with one or more components of the implant 200 (e.g., slots, openings, vents, engagement features, etc.). In some embodiments, the implant 200 may be produced and/or provided (e.g., as part of a sterile surgical kit) with the implant 200 in an engaged position such that the interface feature 242 of the implant 200 is engaged with the interface feature 254 of the instrument 200. In some embodiments, the interface feature 254 of the instrument 250 may include alternate geometries, for example an alternate number of splines equivalent to that of the interface feature of the instrument. Further, in some embodiments the interface features 242, 254 may include alternate complimentary geometries (e.g., torx, hexagonal, etc.).

The implant 200 includes a threading 222 arranged on an outer surface 224 of the implant 200 and extending from a proximal-most portion of the top portion 210 to the bottom portion 230 of the implant. As shown in FIGS. 2-4, the threading 222 is equally spaced (e.g., an equal spacing between threads when viewed from a side direction) along the length of the outer surface 224 of the implant 200. As shown in FIGS. 2-4, the threading 222 is a four-lobed thread, although the threading 222 may have other configurations in alternate embodiments. The implant 200 is further shown to include a plurality of vents (e.g., openings) 226 positioned on the outer surface 224 of the implant 200. As shown in the exemplary embodiment of FIG. 2, the vents 226 extend from the proximal-most portion of the top portion 210 to the distal-most portion of the body 220. The implant 200 as shown includes four vents arranged circumferentially about the outer surface 224 of the implant 200 and substantially equidistant from one another (e.g., at approximately 90-degree increments). The vents 226 are shown to extend from the outer surface 224 of the implant to the inner surface 244 of the implant, thus establishing fluid communication between the outer surface 244 of the implant and the channel 240. It should be noted that the vents 226 are arranged so as not to interfere with the interface feature 242 and that the interior surface 244 of the implant 200 may include alternating vents 226 and splines of the interface feature 242 arranged circumferentially within the channel 240. Further, the vents 226 may be configured to align with the openings 256 of the instrument 250 when in an engaged position. After implantation, the vents 226 are configured to facilitate bone ingrowth thus providing additional fixation within bone and/or soft tissue. In some embodiments, the implant 200 may have greater or fewer than the four vents 226 shown in FIG. 2, where said vents 226 may be arranged alternately (e.g., not equidistant circumferentially from one another) about the outer surface 224 of the implant 200. The threading 222 is shown to extend around the body portion 220 from the bottom portion 230 to the top portion 210, with said threading 222 traversing the vents 226 as shown in FIG. 2.

The bottom portion 230 of the implant 200 is shown to include openings 234 extending laterally (e.g., substantially diametrically) through the bottom portion 230 of the implant. As shown in FIG. 2, the channel 240 terminates proximal relative to the openings 234 in the bottom portion 230 of the implant such that the openings 234 are at least partially defined in the longitudinal direction by an interior portion 236 of the bottom portion 230 of the implant 200. The openings 234 are configured to receive one or more sutures which may be threaded prior to implantation, and furthermore may be threaded prior to engagement of the implant 200 with the instrument 250 (e.g., a surgical kit may include the implant 200 engaged with the instrument 250 with one or more sutures threaded through the openings 234). In some embodiments, the implant 200 may include a single opening 234 or may include three or more openings 234 arranged variously about the bottom portion 230 of the implant 200.

The implant 200 includes a tip 232 arranged at the distal-most portion of the bottom portion 230. As shown in FIG. 2, the tip 232 is substantially rounded but may have alternate geometries in other embodiments. The tip 232 is shown to include a tap feature 236 shown as a square tap in the exemplary embodiment of FIGS. 2-4. The tap feature 236 (see FIG. 4) is configured to facilitate implantation in bone and/or soft tissue (including, for example, especially dense bone) without requiring additional instruments or tools (e.g., a drill to create a pilot hole, etc.). Accordingly, a physician may implant the implant 200 via the tap feature 236 into bone in a time-efficient and instrument-efficient manner (which does not require obtaining and switching between multiple instruments). In some embodiments, the tap feature 236 may include geometries other than the square geometry shown and described with reference to FIG. 4. Further, in some embodiments (and depending on the material of which the implant 200 consists of), the tip 232 adjacent the tap feature 236 may include cutting flutes configured to facilitate implantation (for example, a metal embodiment may include cutting flutes whereas a PEEK embodiments may include a square tap feature 236 as shown).

The implant 200 may have one or more standard lengths, with said length measured from the proximal-most point of the top portion 210 to the distal-most portion of the bottom portion 230. For example, the implant 200 may have a length ranging from 2 mm to 6mm, with various different sized produced and/or provided in a surgical kit in increments of 0.5 mm. Additionally, the embodiment of the implant 200 shown in FIGS. 2-4 may be available in multiple lengths (e.g., as components of a surgical kit or independently) so as to accommodate different applications of implantation into bone and/or soft tissue to facilitate repair and/or reconstruction of said soft tissue. Further, the implant 200 may have a standard diameter and/or may be available in various different diameters so as to accommodate different applications of implantation into bone and/or soft tissue to facilitate repair and/or reconstruction of said soft tissue. Such various diameters may be components of a surgical kit (e.g., a sterile kit including multiple lengths and diameters of the implant 200 as well as other components, for example one or more k-wires, drills, and/or drivers) and/or may be available independently of such a kit. The implant 200 may be formed of a metal (e.g., titanium), or one or more forms of PEEK (e.g., HA Peek, carbon fiber reinforced PEEK, HA/CF PEEK, natural PEEK, etc.) and/or other polymeric soft anchor configurations. In some aspects, the implant 200 may be produced and/or provided with a suture (e.g., those shown and described in the patent applications referenced previously, or other similar sutures and/or suture tapes) threaded through the openings 234. Further, in some aspects the implant 200 may be produced and/or provided (e.g., in a sterile surgical kit) in a pre-loaded state, where the implant 200 is engaged with the instrument 250 via the interface features 244, 250 and a suture as described previously threaded through the implant 200.

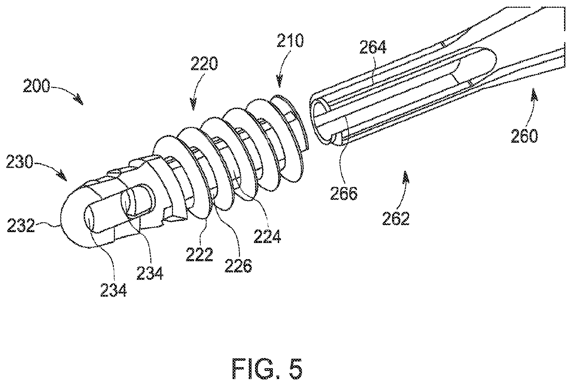

Referring now to FIG. 5, the implant 200 is shown with an instrument 260 for facilitating implantation, manipulation, adjustment, and/or removal of the implant 200. The instrument 260 may have one or more components/features the same as or similar to the instrument 250 as shown and described previously. Further, the instrument 260 is shown to include an interface feature 264 arranged at a distal portion 262 of the instrument 260. The interface feature 264 may have a geometry the same as or similar to that of the interface feature 254, as both interface features 254, 264 are configured to engage with the interface feature 242 of the implant 200. The instrument 260 is further shown to include a recess 266, where the recess 266 includes at least a portion of the cylindrical geometry of the distal portion 262 of the instrument 260. The recess 266 may be configured to facilitate the threading and/or placement of one or more sutures (such as those mentioned previously) and/or needles within/through the recess 266 and/or through one or more elements of the implant 200.

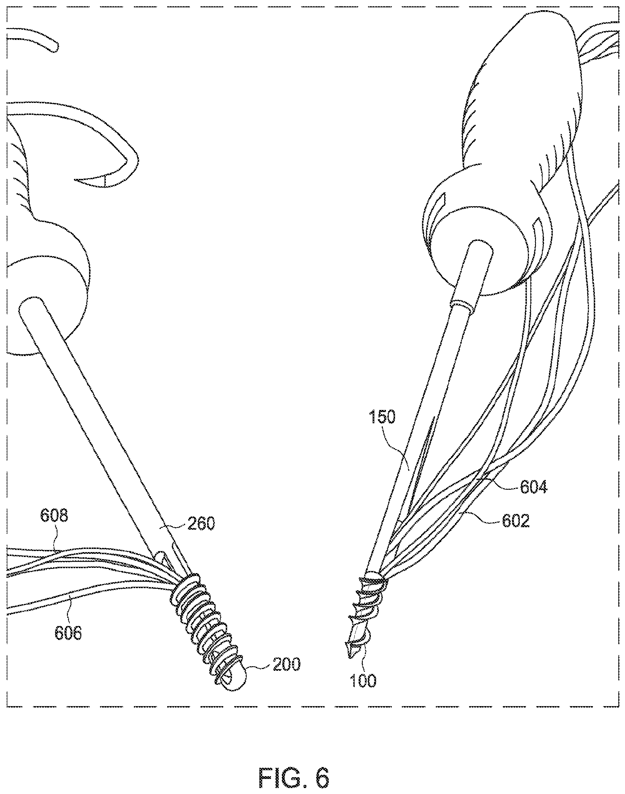

Referring now to FIG. 6, the implant 100 and instrument 150 are shown adjacent the implant 200 and the instrument 260. As shown, the implant 100 is engaged with the instrument 150 via the interface features 114 and 154. Further, a first suture 602 and a second suture 604 are shown to be threaded through the implant 100 and the instrument 150 such that the implant 100 and the instrument 150 are, collectively in a loaded state. As shown, the implant 200 is engaged with the instrument 260 via the interface features 242 and 254. Further, a first suture 606 and a second suture 608 are shown to be threaded through the implant 200 and the instrument 260 such that the implant 200 and the instrument 260 are, collectively in a loaded state. In some aspects, the implant 100 and instrument 150 as well as the implant 200 and the instrument 260 may be produced, provided, and/or sold in the loaded state as shown in FIG. 6.

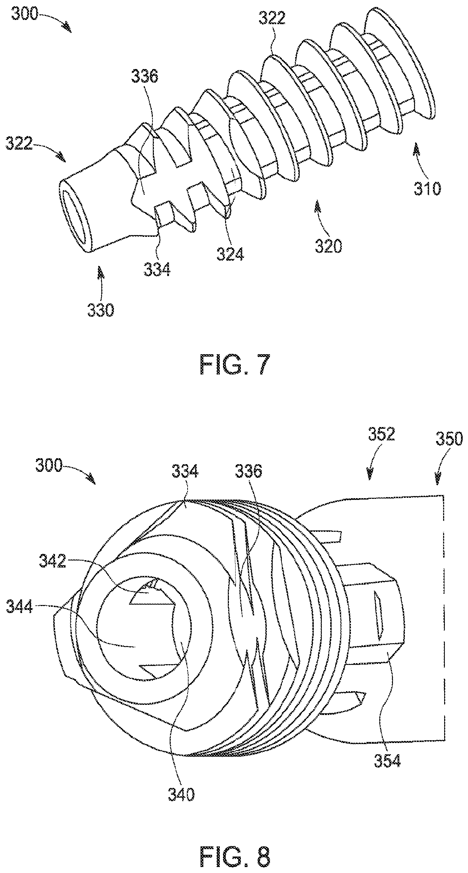

Referring now to FIGS. 7-8, an implant 300 is shown. The implant 300 includes a top portion 310, a bottom portion 330, and a body portion 320 arranged longitudinally between the top portion 310 and the bottom portion 330. The implant 300 is further shown to include a channel (e.g., a cannulated opening, cannulation, etc.) 340 extending along a longitudinal axis of the implant 300 from the proximal-most point of the top portion 310 through the body portion 320 and extending to and through the distal-most point of the bottom portion 330 thus establishing fluid communication in a longitudinal direction from the top portion 310 to and through the bottom portion 330 of the implant 300. The implant 310 is shown to include an interface feature 342 arranged within the channel 340 and, as shown in the exemplary embodiment of FIGS. 7-8, disposed circumferentially about an inner surface 344 of the implant 300. The interface feature 342 of the exemplary embodiment of the implant 300 shown in FIGS. 7-8 includes four splines arranged circumferentially about the inner surface 344 of the implant 300 and extending along the inner surface 344 from the top portion 310 of the implant in a distal direction, with each of said splines positioned in approximately 90-degree increments (e.g., the splines are equally spaced circumferentially about the inner surface 344). In some embodiments, the interface feature 342 may include a greater or lesser number of splines, for example one, two, three, five, or another alternate number of splines. Further, the splines of the interface feature 344 may be arranged variously about the inner surface 344 of the implant 300, for example in some embodiments the splines may not have approximately equal spacing from one another as shown and described in FIGS. 7-8. In some embodiments, the splines may include a geometry other than that shown and described with reference to FIGS. 7-8, for example the splines may include a substantially triangular geometry.

The interface feature 344 is configured to engage (e.g., receive, releasably couple with, etc.) one or more instruments that may be provided in a surgical kit along with the implant 300. As shown in FIGS. 7-8, an instrument (e.g., a driver) 350 is shown to include an interface feature 354 arranged on a distal portion 352 of the instrument 350. The interface feature 354 of the instrument 350 is shown to be complimentary (e.g., geometrically opposite) to that of the interface feature 342. As shown in FIGS. 7-8, the interface feature 354 of the instrument 350 includes four splines complimentary to those of the interface feature 342 of the implant 300. In some embodiments, the complimentary splines of the interface features 342, 354 may be configured to accommodate greater torque than other similar geometries (e.g., torque from the instrument 350 being applied to the implant 300 so as to implant the implant 300 in bone and/or soft tissue). The distal portion 352 of the implant 350 may be received (e.g., to engage, releasably couple, etc.) within the channel 340 of the implant 300 such that the interface feature 352 engages with the interface feature 342 along at least a portion of the length of the interior surface 344 of the channel 340. In some embodiments, the implant 300 may be produced and/or provided (e.g., as part of a sterile surgical kit) with the implant 300 in an engaged position such that the interface feature 342 of the implant 300 is engaged with the interface feature 354 of the instrument 300. In some embodiments, the interface feature 354 of the instrument 350 may include alternate geometries, for example an alternate number of splines equivalent to that of the interface feature of the instrument. Further, in some embodiments the interface features 342, 354 may include alternate complimentary geometries (e.g., torx, hexagonal, etc.). Additionally, in some embodiments the interface features 342, 354 may be configured to provide a press fit between the instrument 350 and the implant 300. In some embodiments, this press fit may include feedback to the user, such as a haptic and/or audible "click" or other feedback to confirm engagement of the press fit feature. Further, the press fit feature may include one or more impressions or depressions arranged circumferentially around the inner surface 344 of the channel 340, with the complimentary (e.g., geometrically opposite) feature arranged circumferentially about the distal portion 352 of the instrument 350.

The implant 300 includes a threading 322 arranged on an outer surface 324 of the implant 300 and extending from a proximal-most portion of the top portion 310 to the distal-most portion of the body 320 of the implant. As shown in FIGS. 7-8, the threading 322 is equally spaced (e.g., an equal spacing between threads when viewed from a side direction) along at least a portion of the length of the outer surface 324 of the implant 300. As shown in FIGS. 7-8, the threading 322 is a three-lobed thread, although the threading 322 may have other configurations in alternate embodiments. The implant 300 includes a plurality of flats arranged on an outer surface 324 of the implant 300, with said flats positioned at the distal-most portion of the body portion 320 and extending to the distal-most portion of the bottom portion 330. As shown, the implant 300 includes four flats 336, although alternate embodiments may have more or fewer flats. The flats 336 are shown to be arranged equidistant from one another about the circumference of the outer surface 324 of the implant but may be arranged alternatively in other embodiments. The implant 300 is further shown to include a tap feature 334 arranged at the proximal-most portion of the bottom portion 330 of the implant 300. As shown in FIGS. 7-8, the tap feature 334 includes a plurality of cutting flutes (e.g., three as shown in FIGS. 7-8, but greater or fewer cutting flutes in alternate embodiments) arranged circumferentially (e.g., in a singular lateral plane) about the outer surface 324 of the implant and substantially equidistant from one another (e.g., approximately 120-degrees from one another). In alternate embodiments, the tap feature 334 may include one or more cutting flutes alternately arranged about the bottom portion 330 of the implant 300. The tap feature 334 is configured to facilitate implantation in bone and/or soft tissue (including, for example, especially dense bone) without requiring additional instruments or tools (e.g., a drill to create a pilot hole, etc.). Accordingly, a physician may implant the implant 300 via the tap feature 334 into bone in a time-efficient and instrument-efficient manner (which does not require obtaining and switching between multiple instruments). In some embodiments, the tap feature 334 may include geometries other than the square geometry shown and described with reference to FIGS. 7-8. The bottom portion 330 of the implant 300 is further show to include a tip 332 arranged at the distal-most portion of the bottom portion 330. The tip 332 is shown to have a substantially cylindrical geometry, with said cylindrical geometry diametrically equal to or lesser than that of the body portion 320 and the top portion 310.

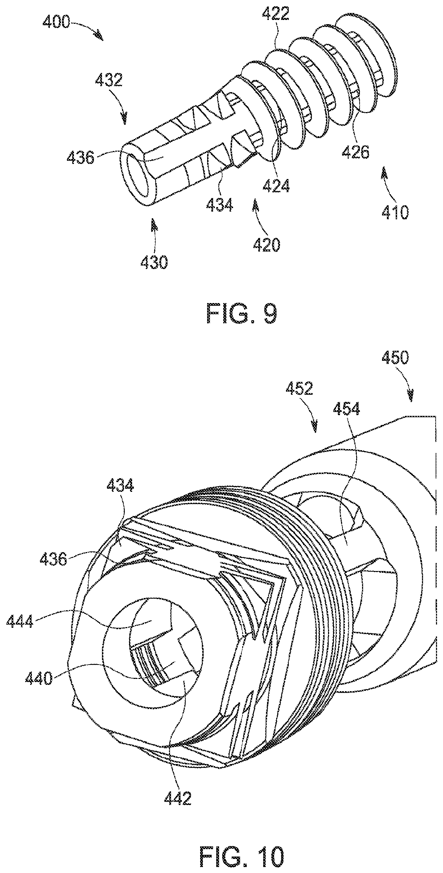

Referring now to FIGS. 9-10, an implant 400 is shown with an instrument 450 for implanting, manipulating, adjusting, and/or removing the implant 100. In some embodiments, the implant 400 may have one or more features the same as or similar to the implant 300 shown and described previously. Further, the instrument 450 may have one or more features the same as and/or similar to the instrument 350 as shown and described previously. The implant 400 includes a top portion 410, a bottom portion 430, and a body portion 420 arranged longitudinally between the top portion 410 and the bottom portion 430. The implant 400 is further shown to include a channel (e.g., a cannulated opening, cannulation, etc.) 440 extending along a longitudinal axis of the implant 400 from the proximal-most point of the top portion 410 through the body portion 420 and extending to and through the distal-most point of the bottom portion 430 thus establishing fluid communication in a longitudinal direction from the top portion 410 to and through the bottom portion 430 of the implant 400. The implant 410 is shown to include an interface feature 442 arranged within the channel 440 and, as shown in the exemplary embodiment of FIGS. 8-9, disposed circumferentially about an inner surface 444 of the implant 400. The interface feature 442 of exemplary embodiment of the implant 400 shown in FIGS. 9-10 includes four splines arranged circumferentially about the inner surface 444 of the implant 400 and extending along the inner surface 444 from the top portion 410 of the implant in a distal direction, with each of said splines positioned in approximately 90-degree increments (e.g., the splines are equally spaced circumferentially about the inner surface 444). In some embodiments, the interface feature 442 may include a greater or lesser number of splines, for example one, two, three, five, or another alternate number of splines. Further, the splines of the interface feature 344 may be arranged variously about the inner surface 444 of the implant 400, for example in some embodiments the splines may not have approximately equal spacing from one another as shown and described in FIGS. 9-10. In some embodiments, the splines may include a geometry other than that shown and described with reference to FIGS. 9-10, for example the splines may include a substantially triangular geometry.

The interface feature 444 is configured to engage (e.g., receive, releasably couple with, etc.) one or more instruments that may be provided in a surgical kit along with the implant 400. As shown in FIGS. 9-10, the instrument (e.g., a driver) 450 is shown to include an interface feature 454 arranged on a distal portion 452 of the instrument 450. The interface feature 454 of the instrument 450 is shown to be complimentary (e.g., geometrically opposite) to that of the interface feature 442. As shown in FIGS. 9-10, the interface feature 454 of the instrument 450 includes four splines complimentary to those of the interface feature 442 of the implant 400. In some embodiments, the complimentary splines of the interface features 442, 454 may be configured to accommodate greater torque than other similar geometries (e.g., torque from the instrument 450 being applied to the implant 400 so as to implant the implant 400 in bone and/or soft tissue). The distal portion 452 of the implant 450 may be received (e.g., to engage, releasably couple, etc.) within the channel 440 of the implant 400 such that the interface feature 452 engages with the interface feature 442 along at least a portion of the length of the interior surface 444 of the channel 440. In some embodiments, the implant 400 may be produced and/or provided (e.g., as part of a sterile surgical kit) with the implant 400 in an engaged position such that the interface feature 442 of the implant 400 is engaged with the interface feature 454 of the instrument 400. In some embodiments, the interface feature 454 of the instrument 450 may include alternate geometries, for example an alternate number of splines equivalent to that of the interface feature of the instrument. Further, in some embodiments the interface features 442, 454 may include alternate complimentary geometries (e.g., torx, hexagonal, etc.). Additionally, in some embodiments the interface features 442, 454 may be configured to provide a press fit between the instrument 450 and the implant 400. In some embodiments, this press fit may include feedback to the user, such as a haptic and/or audible "click" or other feedback to confirm engagement of the press fit feature. Further, the press fit feature may include one or more impressions or depressions arranged circumferentially around the inner surface 344 of the channel 340, with the complimentary (e.g., geometrically opposite) feature arranged circumferentially about the distal portion 452 of the instrument 450.

The implant 400 includes a threading 422 arranged on an outer surface 424 of the implant 400 and extending from a proximal-most portion of the top portion 410 to the distal-most portion of the body 420 of the implant. As shown in FIGS. 9-10, the threading 422 is equally spaced (e.g., an equal spacing between threads when viewed from a side direction) along at least a portion of the length of the outer surface 424 of the implant 400. As shown in FIGS. 9-10, the threading 422 is a four-lobed thread, although the threading 422 may have other configurations in alternate embodiments. The implant 400 includes a plurality of flats arranged on an outer surface 424 of the implant 400, with said flats positioned at the distal-most portion of the body portion 420 and extending to the distal-most portion of the bottom portion 430. As shown, the implant 400 includes four flats 436, although alternate embodiments may have more or fewer flats. The flats 436 are shown to be arranged equidistant from one another about the circumference of the outer surface 424 of the implant but may be arranged alternatively in other embodiments. The implant 400 is further shown to include a tap feature 434 arranged at the proximal-most portion of the bottom portion 430 of the implant 300. As shown in FIGS. 9-10, the tap feature 434 includes a plurality of cutting flutes (e.g., four as shown in FIGS. 9-10, but greater or fewer cutting flutes in alternate embodiments) arranged circumferentially (e.g., in a singular lateral plane) about the outer surface 424 of the implant and substantially equidistant from one another (e.g., approximately 90-degrees from one another). In alternate embodiments, the tap feature 434 may include one or more cutting flutes alternately arranged about the bottom portion 430 of the implant 400. The tap feature 434 is configured to facilitate implantation in bone and/or soft tissue (including, for example, especially dense bone) without requiring additional instruments or tools (e.g., a drill to create a pilot hole, etc.). Accordingly, a physician may implant the implant 400 via the tap feature 434 into bone in a time-efficient and instrument-efficient manner (which does not require obtaining and switching between multiple instruments). In some embodiments, the tap feature 334 may include geometries other than the square geometry shown and described with reference to FIGS. 9-10. The bottom portion 430 of the implant 400 is further show to include a tip 432 arranged at the distal-most portion of the bottom portion 430. The tip 432 is shown to have a substantially cylindrical geometry, with said cylindrical geometry diametrically equal to or lesser than that of the body portion 420 and the top portion 410.

The implant 400 is further shown to include a plurality of vents (e.g., openings) 426 positioned on the outer surface 424 of the implant 400. As shown in the exemplary embodiment of FIG. 9, the vents 426 extend from the proximal-most portion of the top portion 410 to the distal-most portion of the body 420. The implant 400 as shown includes four vents arranged circumferentially about the outer surface 424 of the implant 400 and substantially equidistant from one another (e.g., at approximately 90-degree increments). The vents 426 are shown to extend from the outer surface 424 of the implant to the inner surface 444 of the implant, thus establishing fluid communication between the outer surface 444 of the implant and the channel 440. It should be noted that the vents 426 are arranged so as not to interfere with the interface feature 442 and that the interior surface 444 of the implant 400 may include alternating vents 426 and splines of the interface feature 442 arranged circumferentially within the channel 440. Further, the vents 426 may be configured to align with one or more openings or other components (e.g., recesses) of the instrument 450 when in an engaged position (as shown in FIG. 10). After implantation, the vents 426 are configured to facilitate bone ingrowth thus providing additional fixation within bone and/or soft tissue. In some embodiments, the implant 400 may have greater or fewer than the four vents 426 shown in FIG. 9, where said vents 426 may be arranged alternately (e.g., not equidistant circumferentially from one another) about the outer surface 424 of the implant 400. The threading 422 is shown to extend around the body portion 420 from the bottom portion 430 to the top portion 410, with said threading 422 traversing the vents 426 as shown in FIG. 9.

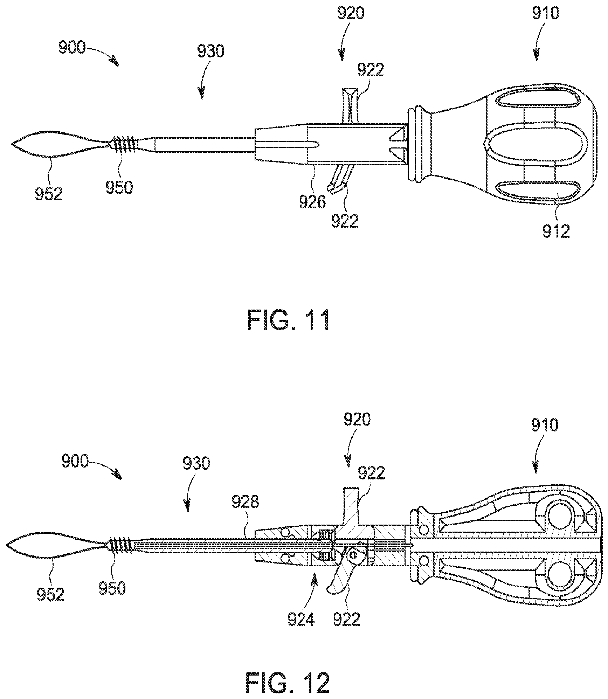

Referring now to FIGS. 11-13, an instrument 900 is shown. The instrument 900 is shown to include a handle 910 and an extension 930 arranged opposite the instrument 900 from the handle. The instrument is further shown to include a body portion 920 arranged between the handle 910 and the extension 930. The handle 910 is shown to have a substantially cylindrical shape with one or more depressions 912 arranged about the surface of the handle 910 (as shown, circumferentially). In some embodiments, the depressions 912 may be configured to facilitate grip of the instrument 900 when applying torque or other force to the instrument 900 (for example, when driving an anchor or implant into soft tissue and/or bone).

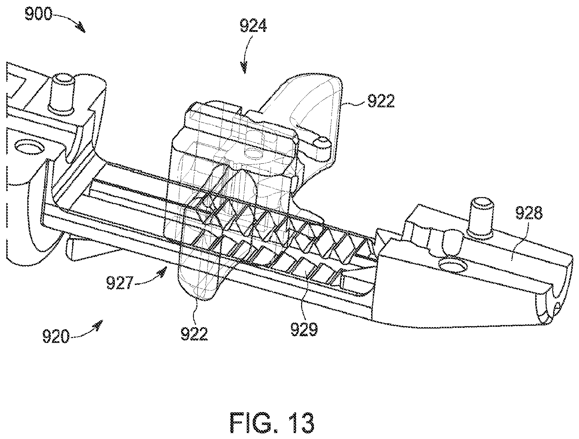

The body portion 920 is shown to include a mechanism 924 and a pair of arms 922, with the arms 922 arranged substantially opposite the body portion 920 one another and extending from the body portion 922 in substantially opposite directions. The mechanism 924 as shown in FIGS. 12-13 is a cam lever mechanism loaded by a resilient member (e.g., a spring), although in other embodiments alternative mechanisms may be implemented. The mechanism 924 is configured within a housing 926. The mechanism 924 is configured to retain (e.g., contact, restrict, hold, etc.) a suture 952 that is threaded through an implant 950. The suture 952 and implant 950 may be the same as or similar to those shown and described previously herein. One of the pair of arms 922 is configured to permit tensioning of the suture 952 by manipulating said arm toward the handle 910. The suture 952 extends from the implant 950 along a track 928 extending substantially along a longitudinal axis of the instrument from the distal-most portion of the extension into the housing 926 of the body portion 920. The mechanism 924 includes a ratchet 927 as shown in FIG. 13 which is configured to facilitate the tensioning of the suture 952 by manipulation of the mechanism 924 via one of the pair of arms 922. The ratchet 929 allows the tensioning of the suture at discrete intervals according to manipulation of the mechanism 924 as the ratchet in manipulated about a set of cam lever teeth 927. The instrument 900 may be configured to hold up to approximately 50 lbf. of tension in the suture 952, which is greater than the estimated tension that a physician could manually apply (approximately 20 lbf). Tensioning of the suture 952 beyond that which is reasonably obtained manually is desirable in repairing and/or reconstructing soft tissue injuries. As the implant 950 is inserted into bone and/or soft tissue by a physician using the instrument 900, the ratchet is overcome and tension of the suture 952 is released. Alternatively, a physician may manually release tension in the suture 952 by manipulating one of the pair of arms 922 toward the implant 900 (e.g., opposite direction of the handle 910).

Any of the implants and/or instruments may be implemented according to the following exemplary technique for a soft tissue repair and/or reconstruction. Additionally, one or more of the steps included herein may be repeated, skipped, or performed in an alternate order in the process of repairing and/or constructing soft tissue.

In performing a soft tissue repair and/or reconstruction, a physician may place a k-wire or other stabilization component corresponding to a first implant to be placed. The physician may then confirm placement of said k-wire using x-ray or other imaging techniques. The physician may then drill or otherwise form a pilot hole corresponding to a desired implant diameter. The physician may then obtain a pre-loaded (e.g., engaged) implant which is engaged with an instrument (where the implant and/or instrument may be threaded with one or more sutures, or the physician may pass a suture through the implant and instrument). The physician may then center the suture tape on the tip of the implant. The physician may then insert the implant. The physician may then prepare an additional pilot hole for an additional implant. The physician may then pass a first portion of a suture from the first implant through a second implant using a suture passer (or the second implant may already be threaded with suture common to the first implant). The physician may then place a portion of the second implant at least partially into the pilot hole. The physician may then manually remove slack from one or more sutures. The physician may then tension the suture between the first implant and second implant using an instrument (e.g., the tensioning instrument shown and described herein). The physician may then insert the implant so as to lock tension of the suture. The physician may then repeat the steps of creating a pilot hole to locking the tension on the suture with a third implant and/or additional implants. When the physician has implanted a desired number of implants and locked in the desired tension of the suture(s), the physician may tie off or trim any excess suture.

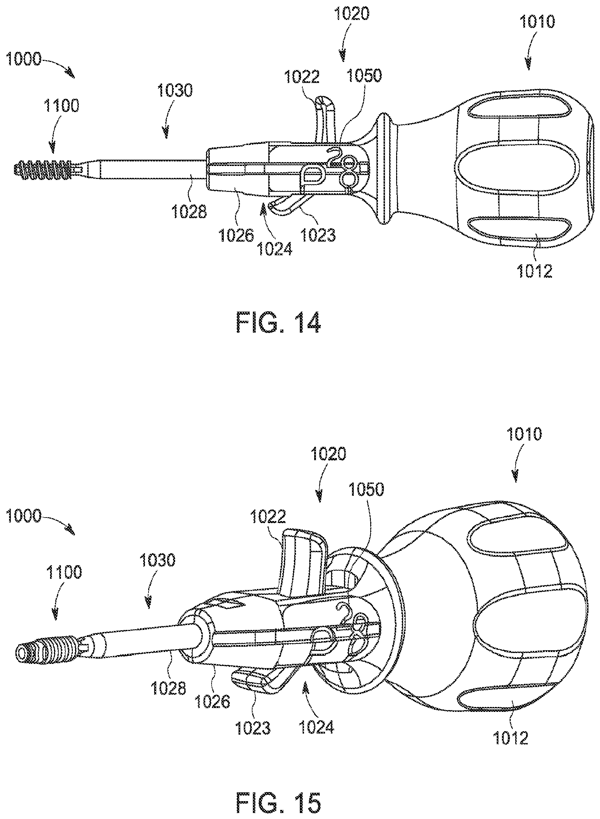

Referring now to FIGS. 14-17, an instrument 1000 is shown. The instrument 1000 is shown to include a handle 1010 and an extension 1030 arranged opposite the instrument 1000 from the handle. The instrument is further shown to include a body portion 1020 arranged between the handle 1010 and the extension 1030. The handle 1010 is shown to have a substantially cylindrical shape with one or more depressions 1012 arranged about the surface of the handle 1010 (as shown, circumferentially). In some embodiments, the depressions 1012 may be configured to facilitate grip of the instrument 1000 when applying torque or other force to the instrument 1000 (for example, when driving an anchor or implant into soft tissue and/or bone).

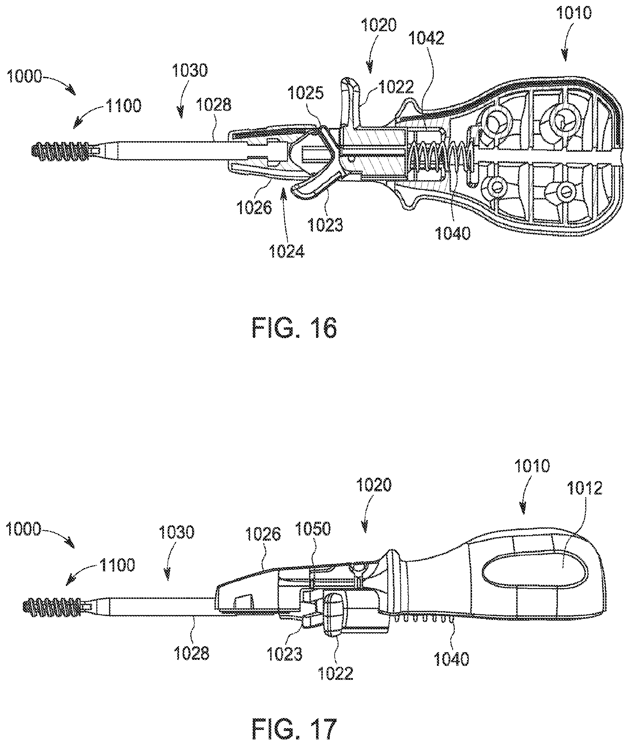

The body portion 1020 is shown to include a mechanism 1024 as well as a first arm 1022 and a second arm 1023, wherein the first and second arms 1022, 1023 are arranged substantially opposite the body portion 1020 one another and extending from the body portion 1022 in substantially opposite directions. As shown in FIG. 16, the second arm 1023 is configured to be pivotable about a coupling point with the first arm 1022. The first arm 1022 may be static and configured to be gripped by a user (e.g., physician) in order to manipulate the instrument 1000. The mechanism 1024 as shown in FIGS. 14-17 is a tensioning mechanism including a resilient member 1040, shown in the exemplary embodiment of FIGS. 14-17 as a spring (although in other embodiments alternative mechanisms may be implemented). The mechanism 1024 is configured without a housing 1026. The mechanism 1024 is configured to retain (e.g., contact, restrict, hold, etc.) a suture (not shown) that is threaded through the implant 1100. Although not shown, the suture may be the same as and/or similar to that shown previously in conjunction with the instrument 900 and the implant 950. The second arm 1023 is shown to be manipulatable (e.g., can be actuated so as to engage the resilient member 1040) by a user so as to engage the mechanism 1024 and components thereof in order to tension a loaded suture. The second arm 1023 is shown to include an engagement portion 1025 configured adjacent a track 1050 (e.g., a continuous depression disposed on at least a portion of an outer surface of the instrument), where the track 1050 is configured to route the suture from the exterior of the instrument 1000 to the interior adjacent the mechanism 1024 (and within the housing 1026). As shown, the track 1050 has a footprint similar to a "Paragon 28" or "P28" logo, but may be modified to other footprints/configurations of various geometries and sizes. Manipulation of the second arm 1023 is shown to retain/release the suture in conjunction with the mechanism 1024 so as to enable tensioning of the suture. In some aspects, the second arm 1023 is configured to be manipulated (while the first arm 1022 is gripped to provide leverage) so as to tension the suture at discrete intervals according to manipulation of the mechanism 1024.

As shown in FIGS. 14-17, the first and second arms 1022, 1023 may be manipulated in a direction toward the handle 1010 so as to drive compression of the resilient member 1040 (and thus releasing tension any tension in the suture). Conversely, when the first and second arms 1022, 1023 are not actuated toward the handle 1010 the suture may be in a tensioned state. The instrument 1000 may be configured to hold up to approximately 50 lbf. of tension in the suture, which is greater than the estimated tension that a physician could manually apply (approximately 20 lbf). Tensioning of the suture beyond that which is reasonably obtained manually is desirable in repairing and/or reconstructing soft tissue injuries. In some aspects, as the implant 1100 is inserted into bone and/or soft tissue by a physician using the instrument 1000, the mechanism 1024 is overcome and tension of the suture may released. Alternatively, a physician may manually release tension in the suture by manipulating The second arm 1023 away from the implant 1100 (e.g., toward the direction of the handle 1010) such that the engagement portion 1025 disengages with the suture.

Any of the implants and/or instruments may be implemented according to the following exemplary technique for a soft tissue repair and/or reconstruction. Additionally, one or more of the steps included herein may be repeated, skipped, or performed in an alternate order in the process of repairing and/or constructing soft tissue.

In performing a soft tissue repair and/or reconstruction, a physician may place a k-wire or other stabilization component corresponding to a first implant to be placed. The physician may then confirm placement of said k-wire using x-ray or other imaging techniques. The physician may then drill or otherwise form a pilot hole corresponding to a desired implant diameter. The physician may then obtain a pre-loaded (e.g., engaged) implant which is engaged with an instrument (where the implant and/or instrument may be threaded with one or more sutures, or the physician may pass a suture through the implant and instrument). The physician may then center the suture tape on the tip of the implant. The physician may then insert the implant. The physician may then prepare an additional pilot hole for an additional implant. The physician may then pass a first portion of a suture from the first implant through a second implant using a suture passer (or the second implant may already be threaded with suture common to the first implant). The physician may then place a portion of the second implant at least partially into the pilot hole. The physician may then manually remove slack from one or more sutures. The physician may then tension the suture between the first implant and second implant using an instrument (e.g., the tensioning instrument shown and described herein). The physician may then insert the implant so as to lock tension of the suture. The physician may then repeat the steps of creating a pilot hole to locking the tension on the suture with a third implant and/or additional implants. When the physician has implanted a desired number of implants and locked in the desired tension of the suture(s), the physician may tie off or trim any excess suture.

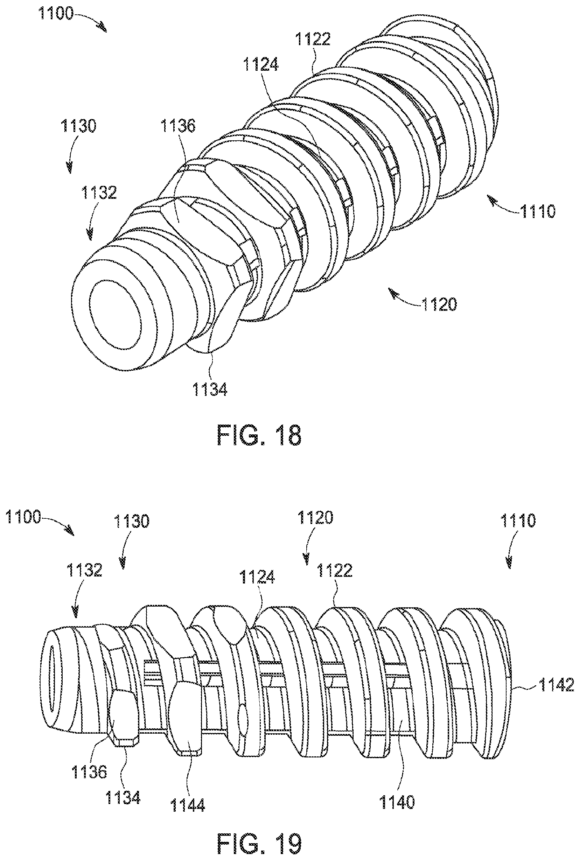

Referring now to FIGS. 18-19, an exemplary embodiment of an implant 1100 is shown. It should be understood that the implant 1100 may have one or more features, geometries, or other properties the same as and/or similar to that of other exemplary implants shown and described previously herein. Similarly, the implant 1100 may be compatible with one or more of the implants and/or instruments shown and described herein (e.g., may be a component of a system including one or more other components shown and described herein). The implant 1100 is shown to include a top portion 1110, a bottom portion 1130, and a body portion 1120 arranged between the top portion 1110 and the bottom portion 1130. The implant 1100 is further shown to include a channel (e.g., a cannulated opening) 1140 extending along a longitudinal axis of the implant 1100 from the proximal-most point of the top portion 1110 through the body portion 1120 and extending through the distal-most point of the bottom portion 1130. The implant 1100 is shown to include an interface feature 1142 arranged within the channel 1140 which may be the same as and/or similar to that of one or more other implants shown and described previously herein, (e.g., the exemplary embodiment of FIGS. 2-4), disposed circumferentially about an inner surface of the implant 1100.

The interface feature 1142 is configured to engage (e.g., receive, releasably couple with, etc.) one or more instruments that may be provided in a surgical kit along with the implant 1100. The implant 1100 may be configured to engage with the instrument 250 shown previously in the same or a similar manner to that of the implant 200. Alternatively, the implant 1100 may be configured to interface with other similar instruments or may also have compatibility with various instruments and/or various common coupling geometries or sizes (e.g., Torx, etc.).

The implant 1100 includes a threading 1122 arranged on an outer surface 1124 of the implant 1100 and extending from a proximal-most portion of the top portion 1110 to the bottom portion 1130 of the implant. As shown in FIGS. 18-19, the threading 1122 is equally spaced (e.g., an equal spacing between threads when viewed from a side direction) along the length of the outer surface 1124 of the implant 1100. The threading 1122 may, in some aspects, be a four-lobed thread, or may have other configurations in alternate embodiments. Similarly, in some aspects the threading 1122 may have a self-tapping configuration. The implant 1100 is further shown to include a plurality of vents (e.g., openings) 1126 positioned on the outer surface 1124 of the implant 1100. As shown in the exemplary embodiment of FIGS. 18-19, the vents 1126 extend from the proximal-most portion of the top portion 1110 to the distal-most portion of the body 1120. The implant 1100 as shown includes the vents 1126 arranged circumferentially about the outer surface 1124 of the implant 200 and substantially equidistant from one another and may vary in quantity and spacing from one another (e.g., at approximately rotational degree increments of 90-degrees, 180-degrees, etc.). The vents 1126 are shown to extend from the outer surface 1124 of the implant 1100 to the inner surface 1144 of the implant, thus establishing fluid communication between the outer surface 1124 of the implant 1100 and the channel 1140. It should be noted that the vents 1126 are arranged so as not to interfere with the interface feature 1142 and that the interior surface 1144 of the implant 1100 may include alternating vents 1126 and splines of the interface feature 1142 arranged circumferentially within the channel 1140. Further, the vents may be configured to accommodate an instrument, for example to align with the openings 256 of the instrument 250 when in an engaged position. After implantation, the vents 1126 are configured to facilitate bone ingrowth thus providing additional fixation within bone and/or soft tissue. In some embodiments, the implant 1100 may have greater or fewer than the four vents 1126 shown in FIGS. 18-19, where said vents 1126 may be arranged alternately (e.g., not equidistant circumferentially from one another) about the outer surface 1124 of the implant 1100. The threading 1122 is shown to extend around the body portion 1120 from the bottom portion 1130 to the top portion 1110, with said threading 1122 traversing the vents 1126.

The implant 1100 includes a tip 1132 arranged at the distal-most portion of the bottom portion 1130. The tip 1132 is shown to have a substantially cylindrical geometry, but may have alternate geometries in other embodiments. The tip 1132 is shown to include a tap feature 1136 shown as a square tap in the exemplary embodiment of FIGS. 18-19. The tap feature 1136 is configured to facilitate implantation in bone and/or soft tissue (including, for example, especially dense bone) without requiring additional instruments or tools (e.g., a drill to create a pilot hole, etc.). Accordingly, a physician may implant the implant 1100 via the tap feature 1136 into bone in a time-efficient and instrument-efficient manner (which does not require obtaining and switching between multiple instruments). In some embodiments, the tap feature 1136 may include geometries other than the square geometry shown and described with reference to FIGS. 18-19. Further, in some embodiments (and depending on the material of which the implant 1100 consists of), the tip 1132 adjacent the tap feature 1136 may include cutting flutes configured to facilitate implantation (for example, a metal embodiment may include cutting flutes, whereas a PEEK embodiments may include a square tap feature 1136 as shown).

The implant 1100 may have one or more standard lengths, with said length measured from the proximal-most point of the top portion 1110 to the distal-most portion of the bottom portion 1130. For example, the implant 1100 may have a length ranging from 2 mm to 6mm, with various different sized produced and/or provided in a surgical kit in increments of 0.5 mm. Additionally, the embodiment of the implant 1100 shown in FIGS. 2-4 may be available in multiple lengths (e.g., as components of a surgical kit or independently) so as to accommodate different applications of implantation into bone and/or soft tissue to facilitate repair and/or reconstruction of said soft tissue. Further, the implant 1100 may have a standard diameter and/or may be available in various different diameters so as to accommodate different applications of implantation into bone and/or soft tissue to facilitate repair and/or reconstruction of said soft tissue. Such various diameters may be components of a surgical kit (e.g., a sterile kit including multiple lengths and diameters of the implant 1100 as well as other components, for example one or more k-wires, drills, and/or drivers) and/or may be available independently of such a kit. The implant 1100 may be formed of a metal (e.g., titanium), or one or more forms of PEEK (e.g., HA Peek, carbon fiber reinforced PEEK, HA/CF PEEK, natural PEEK, etc.) and/or other polymeric soft anchor configurations. In some aspects, the implant 1100 may be produced and/or provided with a suture (e.g., those shown and described in the patent applications referenced previously, or other similar sutures and/or suture tapes) threaded through the openings 1134. Further, in some aspects the implant 1100 may be produced and/or provided (e.g., in a sterile surgical kit) in a pre-loaded state, where the implant 1100 is engaged with the instrument 250 and a suture as described previously threaded through the implant 1100.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has", and "having"), "include" (and any form of include, such as "includes" and "including"), and "contain" (and any form of contain, such as "contains" and "containing") are open-ended linking verbs. As a result, a method or device that "comprises," "has," "includes," or "contains" one or more steps or elements possesses those one or more steps or elements, but is not limited to possessing only those one or more steps or elements. Likewise, a step of a method or an element of a device that "comprises," "has," "includes," or "contains" one or more features possesses those one or more features, but is not limited to possessing only those one or more features. Furthermore, a device or structure that is configured in a certain way is configured in at least that way, but may also be configured in ways that are not listed.

The invention has been described with reference to the preferred embodiments. It will be understood that the architectural and operational embodiments described herein are exemplary of a plurality of possible arrangements to provide the same general features, characteristics, and general system operation. Modifications and alterations will occur to others upon a reading and understanding of the preceding detailed description.

## Claims

1. A system for repairing or reconstructing soft tissue, wherein the system comprises:
an instrument (250, 900, 1000) having a handle (910, 1010) and an extension (930, 1030), the extension (930, 1030) being opposite the handle (910, 1010) and comprising a distal portion (252) with an opening (256) extending laterally through the distal portion (252);
an implant (200), comprising:
a top portion (210);
a bottom portion (230) defining a tip (232) and at least one opening (234) proximally spaced from the tip (232) extending laterally through the bottom portion (230);
a body portion (220)
extending between the top portion (210) and the bottom portion (230);
a cannulated opening (240) extending longitudinally from a proximal end of the top portion (210) and terminating within the body portion (220) proximally of the
at least one opening (234) extending laterally through the bottom portion (230);
threading (222) disposed along an outer surface 224 of the top portion (210) and the body portion (220); and
at least one vent opening (226) disposed in at least one of the top portion (210) and the body portion (220),
the at least one vent opening (226) extending laterally from the outer surface (224) of the top portion (210) or the body portion (220) to an
inner surface (244) at the cannulated opening (240) to establish fluid communication between the outer surface (224) and the cannulated opening (240); and
a suture (602, 604, 606, 608, 952) threaded through the at least one opening (234) extending laterally through the bottom portion (230) of the implant (200), wherein the suture (602, 604, 606, 608, 952) is engaged with a tensioning mechanism (924, 1024) of the handle (910, 1010) of the instrument (250, 900, 1000),
wherein the implant (200) is releasably engaged on the distal portion (252) of the extension (930, 1030) of the instrument (250, 900, 1000) such that the distal portion (252) of the extension (930, 1030) of the instrument (250, 900, 1000) is received within the cannulated opening (240) of the implant (200) and the opening (256) in the distal portion (252) of the extension (930, 1030) is aligned with the at least one vent opening (226) in the implant (200).

2. The system of claim 1, wherein the top portion (200), the bottom portion (230), and the body portion (220) are arranged about a common longitudinal axis.

3. The system of claim 2, wherein the cannulated opening (240) terminates at a distal-most portion of the body portion (220).

4. The system of claim 3, wherein the at least one vent opening (226) comprises a plurality of longitudinally spaced vent openings (226) disposed longitudinally along, and arranged circumferentially about, the outer surface (224) of the top portion (210) or the body portion (220).

5. The system of claim 1, wherein the at least one vent opening (226) is positioned in a valley extending between a pair of adj acent threads of the threading (222).

6. The system of claim 1, wherein the threading (222) spans at least a portion of the at least one vent opening (226).

7. The system of claim 1, wherein the implant (200) further comprises:
at least one tap (236) arranged on an outer surface of the bottom portion (230) of the implant (200).

8. The system of claim 7, wherein the at least one tap (236) comprises a plurality of taps arranged about the outer surface of the bottom portion (230) of the implant (200), wherein the plurality of taps (236) protrude from the outer surface and are equidistantly spaced from one another.

9. The system of claim 1, wherein the opening (256) in the distal portion (252) of the extension (930, 1030) is aligned with a plurality of vent openings (226) of the implant (200).

10. The system of claim 9, wherein the tensioning mechanism (924, 1024) is disposed within a housing of the instrument (250, 900, 1000).

11. The system of claim 10, wherein the tensioning mechanism (924, 1024) comprises:
a first arm (922, 1022);
a second arm (922, 1023) pivotably coupled with the first arm (922, 1022); and
a resilient member (1040).

12. The system of claim 11, wherein the instrument (250, 900, 1000) further comprises:
a track (1050) disposed on at least a portion of the handle (910, 1010), wherein the track (1050) is configured to receive at least a portion of the suture (602, 604, 606, 608, 952) at least partially therein.

13. The system of claim 12, wherein the second arm (922, 1023) comprises an engagement portion (1025) configured to engage with at least a portion of the suture (602, 604, 606, 608, 952), wherein the engagement portion (1025) is arranged adjacent at least a portion of the track (1050).

14. The system of claim 13, wherein the resilient member (1040) is a spring, and actuation of at least one of the first arm (922, 1022) and the second arm (922, 1023) compresses the spring.

15. The system of claim 1, wherein the at least one at least one opening (234) extending through the bottom portion (230) comprises a pair of longitudinally spaced openings (234, 234) that extend through the bottom portion (230) and are longitudinally spaced between the tip (232) and the body portion (220).

## Patentansprüche

1. Ein System zum Reparieren oder Rekonstruieren von Weichteilgewebe, wobei das System Folgendes beinhaltet:
ein Instrument (250, 900, 1000) mit einem Griff (910, 1010) und einer Verlängerung (930, 1030), wobei die Verlängerung (930, 1030) dem Griff (910, 1010) gegenüberliegt und einen distalen Abschnitt (252) mit einer Öffnung (256) beinhaltet, die sich seitlich durch den distalen Abschnitt (252) erstreckt;
ein Implantat (200), das Folgendes beinhaltet:
einen oberen Abschnitt (210);
einen unteren Abschnitt (230), der eine Spitze (232) und mindestens eine Öffnung (234) definiert, die proximal von der Spitze (232) beabstandet ist und sich seitlich durch den unteren Abschnitt (230) erstreckt;
einen Körperabschnitt (220), der sich zwischen dem oberen Abschnitt (210) und dem unteren Abschnitt (230) erstreckt;
eine kanülierte Öffnung (240), die sich in Längsrichtung von einem proximalen Ende des oberen Abschnitts (210) erstreckt und innerhalb des Körperabschnitts (220) proximal von der mindestens einen Öffnung (234) endet, die sich seitlich durch den unteren Abschnitt (230) erstreckt;
ein Gewinde (222), das entlang einer Außenfläche 224 des oberen Abschnitts (210) und des Körperabschnitts (220) angeordnet ist; und
mindestens eine Entlüftungsöffnung (226), die in mindestens einem von dem oberen Abschnitt (210) und dem Körperabschnitt (220) angeordnet ist, wobei sich die mindestens eine Entlüftungsöffnung (226) seitlich von der Außenfläche (224) des oberen Abschnitts (210) oder des Körperabschnitts (220) zu einer Innenfläche (244) an der kanülierten Öffnung (240) erstreckt, um eine Fluidverbindung zwischen der Außenfläche (224) und der kanülierten Öffnung (240) herzustellen; und
ein Nahtmaterial (602, 604, 606, 608, 952), das durch die mindestens eine Öffnung (234) gefädelt ist, die sich seitlich durch den unteren Abschnitt (230) des Implantats (200) erstreckt, wobei das Nahtmaterial (602, 604, 606, 608, 952) mit einem Spannmechanismus (924, 1024) des Griffs (910, 1010) des Instruments (250, 900, 1000) in Eingriff steht,
wobei das Implantat (200) lösbar mit dem distalen Abschnitt (252) der Verlängerung (930, 1030) des Instruments (250, 900, 1000) in Eingriff steht, sodass der distale Abschnitt (252) der Verlängerung (930, 1030) des Instruments (250, 900, 1000) innerhalb der kanülierten Öffnung (240) des Implantats (200) aufgenommen wird und die Öffnung (256) in dem distalen Abschnitt (252) der Verlängerung (930, 1030) auf die mindestens eine Entlüftungsöffnung (226) in dem Implantat (200) ausgerichtet ist.

2. System gemäß Anspruch 1, wobei der obere Abschnitt (200), der untere Abschnitt (230) und der Körperabschnitt (220) um eine gemeinsame Längsachse eingerichtet sind.

3. System gemäß Anspruch 2, wobei die kanülierte Öffnung (240) an einem distalsten Abschnitt des Körperabschnitts (220) endet.

4. System gemäß Anspruch 3, wobei die mindestens eine Entlüftungsöffnung (226) eine Vielzahl von in Längsrichtung beabstandeten Entlüftungsöffnungen (226) beinhaltet, die in Längsrichtung entlang der Außenfläche (224) des oberen Abschnitts (210) oder des Körperabschnitts (220) angeordnet sind und in Umfangsrichtung um diese eingerichtet sind.

5. System gemäß Anspruch 1, wobei die mindestens eine Entlüftungsöffnung (226) in einem Tal positioniert ist, das sich zwischen einem Paar benachbarter Gewindezähne des Gewindes (222) erstreckt.

6. System gemäß Anspruch 1, wobei das Gewinde (222) über mindestens einen Abschnitt der mindestens einen Entlüftungsöffnung (226) verläuft.

7. System gemäß Anspruch 1, wobei das Implantat (200) ferner Folgendes beinhaltet:
mindestens einen Gewindebohrer (236), der auf einer Außenfläche des unteren Abschnitts (230) des Implantats (200) eingerichtet ist.

8. System gemäß Anspruch 7, wobei der mindestens eine Gewindebohrer (236) eine Vielzahl von Zapfen beinhaltet, die um die Außenfläche des unteren Abschnitts (230) des Implantats (200) eingerichtet sind, wobei die Vielzahl von Gewindebohrern (236) von der Außenfläche vorstehen und äquidistant voneinander beabstandet sind.

9. System gemäß Anspruch 1, wobei die Öffnung (256) in dem distalen Abschnitt (252) der Verlängerung (930, 1030) auf eine Vielzahl von Entlüftungsöffnungen (226) des Implantats (200) ausgerichtet ist.

10. System gemäß Anspruch 9, wobei der Spannmechanismus (924, 1024) innerhalb eines Gehäuses des Instruments (250, 900, 1000) angeordnet ist.

11. System gemäß Anspruch 10, wobei der Spannmechanismus (924, 1024) Folgendes beinhaltet:
einen ersten Arm (922, 1022);
einen zweiten Arm (922, 1023), der schwenkbar mit dem ersten Arm (922, 1022) gekoppelt ist; und
ein elastisches Element (1040).

12. System gemäß Anspruch 11, wobei das Instrument (250, 900, 1000) ferner Folgendes beinhaltet:
eine Schiene (1050), die auf mindestens einem Abschnitt des Griffs (910, 1010) angeordnet ist, wobei die Schiene (1050) dazu konfiguriert ist, um mindestens einen Abschnitt des Nahtmaterials (602, 604, 606, 608, 952) mindestens teilweise darin aufzunehmen.

13. System gemäß Anspruch 12, wobei der zweite Arm (922, 1023) einen Eingriffsabschnitt (1025) beinhaltet, der dazu konfiguriert ist, um mit mindestens einem Abschnitt des Nahtmaterials (602, 604, 606, 608, 952) in Eingriff zu stehen, wobei der Eingriffsabschnitt (1025) benachbart zu mindestens einem Abschnitt der Schiene (1050) eingerichtet ist.

14. System gemäß Anspruch 13, wobei das elastische Element (1040) eine Feder ist und die Betätigung von mindestens einem von dem ersten Arm (922, 1022) und dem zweiten Arm (922, 1023) die Feder zusammendrückt.

15. System gemäß Anspruch 1, wobei die mindestens eine Öffnung (234), die sich durch den unteren Abschnitt (230) erstreckt, ein Paar in Längsrichtung beabstandeter Öffnungen (234, 234) beinhaltet, die sich durch den unteren Abschnitt (230) erstrecken und in Längsrichtung zwischen der Spitze (232) und dem Körperabschnitt (220) beabstandet sind.

## Revendications

1. Un système destiné à la réparation ou à la reconstruction de tissu mou, le système comprenant :
un instrument (250, 900, 1000) doté d'une poignée (910, 1010) et d'une extension (930, 1030), l'extension (930, 1030) étant à l'opposé de la poignée (910, 1010) et comprenant une portion distale (252) avec une ouverture (256) s'étendant latéralement à travers la portion distale (252) ;
un implant (200), comprenant :
une portion supérieure (210) ;
une portion inférieure (230) définissant une pointe (232) et au moins une ouverture (234) espacée proximalement de la pointe (232) s'étendant latéralement à travers la portion inférieure (230) ;
une portion formant corps (220) s'étendant entre la portion supérieure (210) et la portion inférieure (230) ;
une ouverture cannelée (240) s'étendant longitudinalement depuis une extrémité proximale de la portion supérieure (210) et se terminant à l'intérieur de la portion formant corps (220) proximalement par rapport à l'au moins une ouverture (234) s'étendant latéralement à travers la portion inférieure (230) ;
un filetage (222) disposé le long d'une surface externe 224 de la portion supérieure (210) et de la portion formant corps (220) ; et
au moins une ouverture formant évent (226) disposée dans au moins une portion parmi la portion supérieure (210) et la portion formant corps (220), l'au moins une ouverture formant évent (226) s'étendant latéralement depuis la surface externe (224) de la portion supérieure (210) ou de la portion formant corps (220) jusqu'à une surface interne (244) au niveau de l'ouverture cannelée (240) afin d'établir une communication fluidique entre la surface externe (224) et l'ouverture cannelée (240) ; et
un fil de suture (602, 604, 606, 608, 952) enfilé à travers l'au moins une ouverture (234) s'étendant latéralement à travers la portion inférieure (230) de l'implant (200), le fil de suture (602, 604, 606, 608, 952) étant engagé avec un mécanisme de tension (924, 1024) de la poignée (910, 1010) de l'instrument (250, 900, 1000),
dans lequel l'implant (200) est engagé de façon libérable sur la portion distale (252) de l'extension (930, 1030) de l'instrument (250, 900, 1000) de telle sorte que la portion distale (252) de l'extension (930, 1030) de l'instrument (250, 900, 1000) est reçue à l'intérieur de l'ouverture cannelée (240) de l'implant (200) et que l'ouverture (256) dans la portion distale (252) de l'extension (930, 1030) est alignée avec l'au moins une ouverture formant évent (226) dans l'implant (200).

2. Le système de la revendication 1, dans lequel la portion supérieure (200), la portion inférieure (230) et la portion formant corps (220) sont agencées autour d'un axe longitudinal commun.

3. Le système de la revendication 2, dans lequel l'ouverture cannelée (240) se termine au niveau d'une portion la plus distale de la portion formant corps (220).

4. Le système de la revendication 3, dans lequel l'au moins une ouverture formant évent (226) comprend une pluralité d'ouvertures formant évents (226) espacées longitudinalement disposées longitudinalement le long de, et agencées circonférentiellement autour de, la surface externe (224) de la portion supérieure (210) ou de la portion formant corps (220).

5. Le système de la revendication 1, dans lequel l'au moins une ouverture formant évent (226) est positionnée dans un creux s'étendant entre une paire de filets adjacents du filetage (222).

6. Le système de la revendication 1, dans lequel le filetage (222) couvre au moins une portion de l'au moins une ouverture formant évent (226).

7. Le système de la revendication 1, dans lequel l'implant (200) comprend en outre :
au moins un taraud (236) agencé sur une surface externe de la portion inférieure (230) de l'implant (200).

8. Le système de la revendication 7, dans lequel l'au moins un taraud (236) comprend une pluralité de tarauds agencés autour de la surface externe de la portion inférieure (230) de l'implant (200), dans lequel la pluralité de tarauds (236) font saillie depuis la surface externe et sont espacés de manière équidistante les uns des autres.

9. Le système de la revendication 1, dans lequel l'ouverture (256) dans la portion distale (252) de l'extension (930, 1030) est alignée avec une pluralité d'ouvertures formant évents (226) de l'implant (200).

10. Le système de la revendication 9, dans lequel le mécanisme de tension (924, 1024) est disposé à l'intérieur d'un boîtier de l'instrument (250, 900, 1000).

11. Le système de la revendication 10, dans lequel le mécanisme de tension (924, 1024) comprend :
un premier bras (922, 1022) ;
un deuxième bras (922, 1023) couplé de façon pivotante au premier bras (922, 1022) ; et
un élément élastique (1040).

12. Le système de la revendication 11, dans lequel l'instrument (250, 900, 1000) comprend en outre :
une glissière (1050) disposée sur au moins une portion de la poignée (910, 1010), la glissière (1050) étant configurée pour recevoir au moins une portion du fil de suture (602, 604, 606, 608, 952) au moins partiellement dans celle-ci.

13. Le système de la revendication 12, dans lequel le deuxième bras (922, 1023) comprend une portion d'engagement (1025) configurée pour s'engager avec au moins une portion du fil de suture (602, 604, 606, 608, 952), la portion d'engagement (1025) étant agencée de manière adjacente à au moins une portion de la glissière (1050).

14. Le système de la revendication 13, dans lequel l'élément élastique (1040) est un ressort, et l'actionnement d'au moins un bras parmi le premier bras (922, 1022) et le deuxième bras (922, 1023) comprime le ressort.

15. Le système de la revendication 1, dans lequel l'au moins une ouverture (234) s'étendant à travers la portion inférieure (230) comprend une paire d'ouvertures espacées longitudinalement (234, 234) qui s'étendent à travers la portion inférieure (230) et sont espacées longitudinalement entre la pointe (232) et la portion formant corps (220).
